Europäisches Patentamt

European Patent Office

Office européen des brevets

(11) Publication number: **0 196 075**
**B1**

# EUROPEAN PATENT SPECIFICATION

(45) Date of publication of patent specification: **14.03.90**

(21) Application number: **86104114.3**

(22) Date of filing: **25.03.86**

(51) Int. Cl.⁵: **C 11 D 7/54,** G 02 C 13/00,
A 61 L 2/16, C 11 D 7/10,
C 11 D 7/08, C 11 D 7/26,
G 02 C 7/04

(54) Cleaning system for contact lenses and process for cleaning the same.

(30) Priority: **26.03.85 JP 61599/85**
**15.05.85 JP 101424/85**

(43) Date of publication of application:
**01.10.86 Bulletin 86/40**

(45) Publication of the grant of the patent:
**14.03.90 Bulletin 90/11**

(84) Designated Contracting States:
**DE FR GB IT**

(56) References cited:
**EP-A-0 175 826**
**WO-A-85/04107**
**BE-A- 865 879**
**GB-A-2 019 600**
**GB-A-2 072 371**
**US-A-3 873 696**

**PATENT ABSTRACTS OF JAPAN, vol. 7, no. 93
(P-192)(1238), 19th April 1983; & JP - A - 58 018
617 (TORAY K.K.) 03-02-1983 (Cat. D)**

(73) Proprietor: **TORAY INDUSTRIES, INC.
2, Nihonbashi-Muromachi 2-chome Chuo-ku
Tokyo 103 (JP)**

(72) Inventor: **Kenjo, Hideki
4-19-3 Sonoyama 2-cho.
Otsu-shi Shiga-ken, 520 (JP)**
Inventor: **Jyono, Teruo
15-51 Oaza Izumi Minakuchi-cho
Kouga-gun Shiga-ken (JP)**

(74) Representative: **Grams, Klaus Dieter, Dipl.-Ing.
et al
Patentanwaltsbüro Tiedtke-Bühling-Kinne-
Grupe-Pellmann-Grams-Struif Winter-Roth
Bavariaring 4
D-8000 München 2 (DE)**

Courier Press, Leamington Spa, England.

**Description**

The present invention relates to a cleaning agent for soft and hard contact lenses (hereinafter referred to as CL) and a process for using the same.

It has been known to clean CL by removing oils, fats and dirts from the surface of CL with a cleaning solution containing, for example, an acid, a chlorite and a surfactant (JP—A—58 018 617 and JP—A—58 018 616). It has also been known that substances such as proteins which cannot be removed with a surfactant or the like are removed by decomposing them with protease.

However, the cleaning solutions used heretofore are those of a two-pack type. In using these solutions, a troublesome operation is required of the users, namely, the two liquids must be measured separately and then placed in a cleaning vessel. Further, containers of a large volume are necessitated for the two liquids. This is inconvenient for a traveler making a trip for a long period of time.

After intensive investigations made for the purpose of overcoming the defects of the conventional cleaning solutions for contact lenses and processes for cleaning them, the inventors have developed a cleaning agent of a two-pack type in which one pack comprises an easily measurable solid and a process for cleaning contact lenses. The cleaning agent can be kept compact as a whole and used easily and conveniently for cleaning contact lenses.

A first embodiment of this invention is directed to a cleaning system for contact lenses, comprising:

a) 0,0001 to 5% by weight of a chlorite salt in aqueous solution, capable of decomposition to form free oxygen for removing impurities from a contact lens;

b) a solid component, comprising:

an agent for accelerating the decomposition of the chlorite to form free oxygen comprising at least one member, selected from the group consisting of acids, organic acid salts, ion exchange resins, reducing agents and sugars; and

an oxygen-consuming agent for consuming excess free oxygen from the decomposition of the chlorite after impurities have been removed from a contact lens being cleaned; and

polyvinylpyrrolidone as a decomposition inhibitor for said agent for accelerating the decomposition of the chlorite.

A second embodiment of the invention provides a process for cleaning contact lenses, comprising placing a lens to be cleaned, and a cleaning system comprising:

a) 0,0001 to 5% by weight of a chlorite salt in aqueous solution, capable of decomposition to form free oxygen for removing impurities from a contact lens;

b) a solid component, comprising:

an agent for accelerating the decomposition of the chlorite to form free oxygen comprising at least one member, selected from the group consisting of acids, organic acid salts, ion exchange resins, reducing agents and sugars; and

an oxygen-consuming agent for consuming excess free oxygen from the decomposition of the chlorite after impurities have been removed from a contact lens being cleaned; and

polyvinylpyrrolidone as a decomposition inhibitor for said agent for accelerating the decomposition of the chlorite in a vessel.

A third embodiment of the invention provides a cleaning system for contact lenses, comprising:

a) 0,0001 to 5% by weight of a chlorite salt in aqueous solution, capable of decomposition to form free oxygen for removing impurities from a contact lens;

b) a solid component, comprising:

an agent for accelerating the decomposition of the chlorite to form free oxygen;

a sugar for consuming excess free oxygen from the decomposition of the chlorite after impurities are removed from the lens; and

polyvinylpyrrolidone as a decomposition inhibitor for the acid.

A further aspect of the invention is directed to a process for cleaning contact lenses, comprising placing a lens to be cleaned and a cleaning system comprising

a) 0,0001 to 5% by weight of a chlorite salt in aqueous solution, capable of decomposition to form free oxygen for removing impurities from a contact lens;

b) a solid component, comprising:

an agent for accelerating the decomposition of the chlorite to form free oxygen;

a sugar for consuming excess free oxygen from the decomposition of the chlorite after impurities are removed from the lens; and

polyvinylpyrrolidone as a decomposition inhibitor for the acid in a vessel.

Yet another aspect of the invention provides a contact lens cleaning kit, comprising:

a) a container with 0,0001 to 5% by weight of a chlorite salt in aqueous solution, capable of decomposition to form free oxygen for removing impurities from a contact lens; and

b) a plurality of packages containing pre-measured amounts of:

an agent for accelerating the decomposition of the chlorite to form free oxygen, selected from the group consisting of acids, organic acid salts, ion exchange resins, reducing agents and sugars; and

an oxygen-consuming agent for consuming excess free oxygen from the decomposition of the chlorite after impurities have been removed from a contact lens being cleaned; and

EP 0 196 075 B1

polyvinylpyrrolidone as a decomposition inhibitor for the agent for accelerating the decomposition of the chlorite; and

c) instructions for using the kit to clean a contact lens.

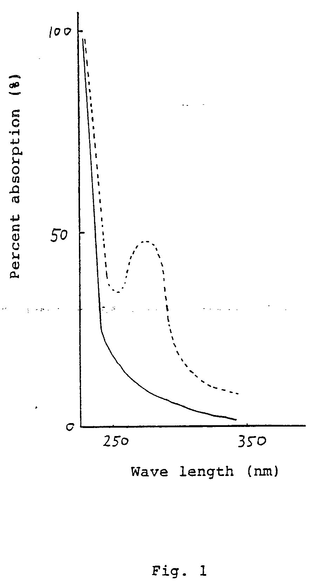

Figure 1 is a chart showing UV spectra of a lens, wherein the dotted line shows the UV spectrum of the stained lens before cleaning and the solid line shows that of the lens treated with cleaning agent of the present invention.

Examples of the chlorites contained in the liquid (a) include sodium and potassium chlorites. Among them, sodium chlorite is particularly preferred, since it effectively decomposes excretions from the living body which are deposited on the contact lenses with minimal effect on the lenses.

Specifically, it is believed that the chlorite ion decomposes to release free oxygen, which attacks the impurities on the lens.

The concentration of the chlorite in the cleaning solution is in the range of 0.0001 to 5% by weight, preferably 0.0005 to 0.5%. For storage stability, a pH in the range of about 6—8 is preferred for the chlorite solution.

In the present invention, the component (b) should be in a solid form so as to facilitate the handling thereof. The form of the solid component (b) is not particularly limited and it may be in the form of tablet, powder or pellet (including granule). Among them, pellet or powder is particularly effective. It is preferred to pack a batch of the component in a bag.

The component (b) must comprise at least one member selected from the group consisting of acids, organic acid salts, ion exchange resins, reducing agents and sugars.

These agents act as a catalyst for accelerating the decomposition of the chlorite contained in the component (a). The agents may function by releasing hydrogen ion, which leads to accelerated decomposition of the chlorite to form free oxygen.

The sugar may be any one having the above-mentioned function and it may be selected from monosaccharides and polysaccharides. A typical example of the sugar is glucose.

The acid may be any one as long as it provides the function required in the present invention. Preferred examples of the acids include organic acids such as adipic, stearic, sebacic, oxalic, itaconic, edetic and ascorbic acid; and inorganic acids such as hydrochloric and sulfuric acids. It is more preferred to use tartaric and/or citric acid. Acids which are normally liquid can be used in a solution form through adsorption on a substrate such as alumina or encapsulation in a suitable acid resistant polymer. In the case of absorption, the chlorite solution itself will release the acid, while some mechanical force may be necessary in the case of encapsulation. The ion exchange resin is preferably a cation exchange resin. The ion exchange resin functions as the acid does, and has the advantage of being in solid form, and thus easily removed after the cleaning is finished. The organic acid salt is preferably at least one of sodium hydrogen-tartrate and sodium dihydrogencitrate. Polyfunctional acid salts are preferred because of the removable acid functions in such salts, which provide the salts with the same properties as an acid with respect to the present invention. Also, such salts often are conveniently available in solid form. The reducing agent is preferably sodium hydrogensulfite.

These compounds accelerate the decomposition of the chlorite.

The pH value of the cleaning solution may be selected suitably so far as the purpose of the present invention is not damaged. It is, for example, in the range of 2 to 8, preferably 3 to 6.

The cleaning agent of the present invention also may contain known additives which do not interfere with the invention, such as: sodium monohydrogenphosphate, urea, boric acid, sodium borate, and edetic acid salts as well as surfactants. These function as preservatives, protein denaturants, etc.

The component (b) may comprise at least one sugar, an acid and a decomposition inhibitor for the acid.

The combination of the sugar with the acid acts as a catalyst for accelerating the decomposition of the chlorite.

The sugar may be any one having the above-mentioned function and it may be selected from among monosaccharides and polysaccharides. Examples of the sugars include glucose, sucrose and fructose. Among them, glucose is particularly preferred. The sugar releases hydrogen ions under the influence of the acid, thus contributing to the decomposition of the chlorite. Furthermore, the sugar function to consume excess free oxygen remaining after the decomposition of impurities so that the excess free oxygen is not available to deteriorate the lens. This latter function of the sugar can be accomplished using other materials so long as the free oxygen attacks the lens impurities before being consumed. An example of a suitable substitute material is a polymer such as polyvinylpyrrolidone. Such a polymer should not be used in the event the lens itself includes the polymer.

The acid may be any one so far as it has the function intended in the present invention. Preferred examples of the acids include organic acids such tartaric, citric, lactic, malic and gluconic acids; and inorganic acids such as hydrochloric and sulfuric acids. It is more preferred to use tartaric and/or citric acid.

A preferred combination of the sugar with the acid comprises citric acid and glucose, since it exhibits an excellent cleaning effect without damaging the physical properties of the lenses. They may be used either alone or in the form of a mixture of two or more of them. The sugar alone will provide some acceleration of the chlorite decomposition, but the efficiency is increased by also using acid.

The present invention is characterized in that a decomposition inhibitor for the acid is contained in the component (b). When a solid mixture of the acid and the sugar is stored for a long period of time, the acid frequently is decomposed, which lowers the cleaning power unfavorably. Though this defect can be prevented by granulating the acid and the sugar separately from each other, this technique is troublesome. According to the present invention, this defect can be overcome by using the decomposition inhibitor. The decomposition inhibitor used in the present invention is polyvinylpyrrolidone. The molecular weight of the polyvinylpyrrolidone is not particularly limited and it ranges from several thousands to several millions.

The cleaning agent of the present invention can be used for cleaning conventional contact lenses to exhibit not only a protein-removing effect but also a decolorizing effect within a short treatment time.

A preferred embodiment of the process for cleaning contact lenses with the cleaning agent of the present invention is as follows: a given amount (for example, a batch packed in a bag) of the solid component (b) is placed in a vessel together with the lenses and then a given amount (for example, corresponding to a scale mark of the cleaning vessel) of the liquid (a) is added thereto to clean the lenses.

The temperature of the cleaning solution may be selected suitably. More particularly, it may be room temperature or from around a boiling point to 120°C. The lower the temperature, the longer the treatment time and, on the contrary, the higher the former, the shorter the latter.

The contact lenses thus treated are preferably left to cool to room temperature in the cleaning solution and then washed with water. If necessary, any remaining chlorite is decomposed with sodium thiosulfate or sodium hydrogensulfite. The lenses are then immersed in a physiological saline and used again.

The contact lenses which can be treated by the above-mentioned cleaning process include, for example, highly hydrous, soft contact lenses and lowly hydrous soft contact lenses comprising mainly polyhydroxyethyl methacrylate. Further, hard contact lenses having a polysiloxane bond, oxygen-permeable contact lenses having both siloxane and urethane bonds, etc. may also be treated as a matter of course. Thus, the kinds of the contact lenses which can be treated according to the present invention are not limited.

The cleaning agent of the present invention in which one of the components is in solid form has advantages in that it can be weighed esily at the time of the use and it can be stored compact as a whole. The present invention has advantageous effects in that it provides a convenient cleaning agent and process for the users.

Another effect of the invention is that the cleaning agent can be stored for a long period of time and that the cleaning can be effected in a consistent manner.

The cleaning agent can be sold in a kit form containing a number of pre-measured units of the solid component (preferably individual package), a container with chlorite solution and instructions for use. Optionally, the kit can include a cleaning container for lenses, marked to indicate the appropriate level for chlorite solution addition.

### Example 1

12 g of polyvinylpyrrolidone having an average molecular weight of about 360,000 was added to a mixture of 80 g of D-(+)-glucose and 8 g of L-(+)-tartaric acid. 5 g of pure water was added thereto and the mixture was stirred well and granulated. The granules were dried at 60°C for 1 h and then heat-treated at 40°C for 80 h. The tartaric acid content was measured according to ion chromatography before and after the heat treatment to reveal that they were 7.78% and 7.89%, rfespectively. The difference between them was thus insignificant. This fact suggests that the product has a good storage stability. 4 ml of 100 ppm aqueous sodium chlorite solution was added to 0.1 g of the granules. A used, stained soft contact lens was immersed therein and boiled at 100°C for 15 min. Then, it was left to cool to room temperature. The lens was washed with running pure water for 5 min and its UV spectrum was examined to evaluate the cleaning effects. The results are shown in Table 1. Satisfactory cleaning effects were obtained.

The accompanying figure is a chart showing UV spectra of the lens in which the dotted line shows the UV spectrum of the lens before cleaning and the solid line shows that after cleaning. The peak in the dotted line shows the presence of contaminant.

### Example 2

Cleaning solution No. 2 as shown in Table 1 was prepared by granulation and pulverization effected in the same manner as in Example 1. A used, stained contact lens was immersed in the solution and boiled at 100°C for 15 min or left to stand at room temperature for 16 h. The lens was washed with running water for 5 min and the UV spectrum thereof was examined to evaluate the cleaning effects. The results are shown in Table 1.

The cleaning effect was determined through the degree of staining of the lens placed on a colorimetric dish and was ranked visually as follows:

| Rank | Degree of staining of the lens |
|------|-------------------------------|
| 4 | The staining and discoloration were serious |
| 3 | The staining and discoloration were moderate |
| 2 | The staining and discoloration were slight |
| 1 | The staining and discoloration were negligible |

TABLE 1

| Cleaning solution No.<br>(Composition of the solution) | 1 | 2 |
|---|---|---|
| Component (a) | | |
|   Sodium chlorite | 0.01% | 0.01% |
|   Pure water | 99.99 | 99.99 |
| Amount of Component (a) | 4 g | 4 g |
| Component (b) | | |
|   Glucose | 80% | 80% |
|   Tartaric acid | 8% | 8% |
|   Polyvinylpyrrolidone | 12% | 12% |
|   (Average molecular weight<br>  of polyvinylpyrrolidone) | 360,000 | 25,000 |
| Amount of component (b) | 0.1 g | 0.1 g |
| (Treatment conditions) | | |
|   Temperature | 100°C | room temp. |
|   Time | 15 min | 16 h |
| (Cleaning effect) | 1 | 2 |

Example 3

12 g of polyvinylpyrrolidone having an average molecular weight of about 25,000 was added to a mixture of 80 g of D-(+)-glucose and 8 g of L-(+)-tartaric acid and the mixture was stirred thoroughly. 4 ml of 100 ppm sodium chlorite solution was added to 0.1 g of the obtained powder. Further, 1 ml of an aqueous urea solution prepared according to a formulation shown below was added to the mixture. A used, stained contact lens was immersed therein and left to stand at room temperature for 16 h. Thereafter, the lens was taken out and washed with running pure water for 5 min. The cleaning effect was determined by a visual method wherein the lens placed in a colorimetric dish was observed before and after the cleaning. The results are shown in Table 2. Excellent cleaning effects were obtained.

# EP 0 196 075 B1

| | |
|---|---|
| Aqueous urea solution: polyoxyethylene sorbitan monolaurate | 0.3% |
| polyoxyethylene/polyoxypropylene | 0.3% |
| sodium carboxymethylcellulose | 0.02% |
| potassium sorbate | 0.005% |
| urea | 1.0% |
| pure water | the balance |

TABLE 2

| Cleaning solution No. (Composition of the solution) | 3 | 4 |
|---|---|---|
| **Component (a)** | | |
| Sodium chlorite (%) | 0.01 | 0.01 |
| Pure water (%) | 99.99 | 99.99 |
| Amount of component (a) (g) | 4 | 4 |
| **Component (b)** | | |
| Glucose (%) | 80 | 80 |
| Tartaric acid (%) | 8 | 8 |
| Polyvinylpyrrolidone (%) | 12 | 12 |
| (Average molecular weight of polyvinylpyrrolidone) ($\times$ 10,000) | 2.5 | 2.5 |
| Amount of component (b) (g) | 0.01 | 0.01 |
| **Component (c)** | | |
| Polyoxyethylene sorbitan monolaurate (%) | — | 0.3 |
| Polyoxyethylene/polyoxypropylene block polymer (%) | — | 0.3 |
| Sodium carboxymethylcellulose (%) | — | 0.02 |
| Potassium sorbate (%) | — | 0.05 |
| Urea (%) | — | 1.0 |
| Pure water (%) | — | 98.375 |
| Amount of component (c) (g) | — | 1 |
| **(Treatment conditions)** | | |
| Temperature | room temp | room temp |
| Time (h) | 16 | 16 |
| **(Cleaning effect)** | | |
| Before cleaning | 4 | 4 |
| After cleaning | 3 | 2 |

# EP 0 196 075 B1

### Comparative Example 1

A granular component (b) was prepared in the same manner as in Example 1 except that the polyvinylpyrrolidone used as the decomposition inhibitor for the sugar was not added. More particularly, 98 g of D-(+)-glucose was mixed with 2 g of L-(+)-tartaric acid. 17 g of pure water was added to the mixture and the obtained mixture was granulated. The granules were dried at 60°C for 1 h and then heat-treated at 40°C for 80 h. The tartaric acid content of the heat treated granules was analyzed according to ion chromatography to reveal that it was reduced by about 16% from 1.97% to 1.65% by the heat treatment. The product had thus a poor stability. The decomposition products of the acid may not be safe to humans.

When the obtained cleaning agent was used for cleaning a contact lens, only a poor cleaning effect was obtained.

**Claims**

1. A cleaning system for contact lenses comprising:

(a) 0,0001 to 5% by weight of a chlorite salt in aqueous solution, capable of decomposition to form free oxygen for removing impurities from a contact lens;

(b) a solid component, comprising:

an agent for accelerating the decomposition of the chlorite to form free oxygen comprising at least one member, selected from the group consisting of acids, organic acid salts, ion exchange resins, reducing agents and sugars; and

an oxygen-consuming agent for consuming excess free oxygen from the decomposition of the chlorite after impurities have been removed from a contact lens being cleaned; and

polyvinylpyrrolidone as a decomposition inhibitor for said agent for accelerating the decomposition of the chlorite.

2. The system of claim 1, wherein the solid component is in the form of a pellet or powder.

3. The system of claim 1, wherein the agent for accelerating the decomposition is at least one organic acid.

4. The system of claim 1, wherein the agent for accelerating the decomposition is a cation exchange resin.

5. The system of claim 1, wherein the agent for accelerating the decomposition is at least one member selected from the group consisting of adipic, stearic, sebacic, oxalic, itaconic, edetic, ascorbic, alginic, aspartic, sorbic, tartaric and citric acids.

6. The system of claim 1, wherein the agent for accelerating the decomposition is at least one member selected from the group consisting of sodium hydrogentartarate and sodium dihydrogencitrate.

7. The system of claim 1, wherein the agent for accelerating the decomposition is sodium hydrogensulfite.

8. The system of claim 1, wherein the agent for accelerating the decomposition is glucose.

9. The system of claim 1, wherein one of the members of the solid component is both an agent for accelerating the decomposition of chlorite and an oxygen-consuming agent.

10. The system of one of claims 1, 2 or 5, wherein the solid component comprises:

an acid for accelerating decomposition of the chlorite to form free oxygen;

a sugar for consuming excess free oxygen from the decomposition of the chlorite after impurities are removed from the lens; and

polyvinylpyrrolidone as a decomposition inhibitor for the acid.

11. The system of claim 10, wherein the sugar is at least one member selected from the group consisting of glucose, sucrose and fructose.

12. A process for cleaning contact lenses, comprising placing a lens to be cleaned, and a cleaning system according to claim 1 in a vessel.

13. The process of claim 12, wherein a solid component, comprising:

an acid for accelerating decomposition of the chlorite to form free oxygen;

a sugar for consuming excess free oxygen from the decomposition of the chlorite after impurities are removed from the lens; and

polyvinylpyrrolidone as a decomposition inhibitor for the acid is placed in the vessel.

14. The process of claim 12, wherein one member of the solid component acts both as an agent for accelerating the decomposition of chlorite and as an oxygen-consuming agent.

15. A contact lens cleaning kit, comprising

(a) a container with 0,0001 to 5% by weight of a chlorite salt in aqueous solution, capable of decomposition to form free oxygen form removing impurities from a contact lens; and

(b) a plurality of packages containing pre-measured amounts of:

an agent for accelerating the decomposition of the chlorite to form free oxygen, comprising at least one member selected from the group consisting of acids, organic acid salts, ion exchange resins, reducing agents and sugars; and

an oxygen-consuming agent for consuming excess free oxygen from the decompostion of the chlorite after impurities have been removed from a contact lens being cleaned; and

polyvinylpyrrolidone as a decomposition inhibitor for the agent for accelerating the decomposition of

7

the chlorite; and

(c) instructions for using the kit to clean a contact lens.

16. The kit of claim 15, wherein the agent for accelerating the decomposition is an acid and the oxygen-consuming agent is a sugar.

17. The kit of claim 16, wherein the acid is selected from the group consisting of adipic, stearic, sebacic, oxalic, itaconic, edetic, ascorbic, alginic, aspartic, sorbic, tartric and citric acids.

18. The kit of claim 16, wherein the sugar is at least one member selected from the group consisting of glucose, sucrose and fructose.

19. The kit of claim 15, wherein one of the packages comprises a member being both an agent for accelerating the decomposition and an oxygen-consuming agent.

20. The kit of claim 15 further comprising a cleaning vessel for accepting a contact lens to be cleaned.


**Patentansprüche**

1. Reinigungssystem für Kontaktlinsen, bestehend aus:

(a) 0,0001 bis 5 Masse% eines Chloritsalzes in wäßriger Lösung, das unter Bildung von freiem Sauerstoff zur Entfernung von Verunreinigungen von einer Kontaktlinse zesetzbar ist; und

(b) einer festen Komponente, bestehend aus:

einem Mittel zum Beschleunigen der Zersetzung des Chlorits unter Bildung von freiem Sauerstoff, das aus wenigstens einem Bestandteil besteht, der aus der Gruppe ausgewählt ist, die aus Säuren, Salzen organischer Säuren, Ionenaustauscherharzen, Reduktionsmitteln und Zuckern besteht; und

einem sauerstoffverbrauchenden Mittel zum Verbrauchen von überschüssigem freiem Sauerstoff aus der Zersetzung des Chlorits, nachdem von einer Kontaktlinse, die gereinigt wird, Verunreinigungen entfernt worden sind; und

Polyvinylpyrrolidon als Mittel zum Inhibieren der Zersetzung des Mittels zum Beschleunigen der Zersetzung des Chlorits.

2. System nach Anspruch 1, bei dem die feste Komponente in Form einer Tablette oder eines Pulvers vorhanden ist.

3. System nach Anspruch 1, bei dem das Mittel zum Beschleunigen der Zersetzung wenigstens eine organische Säure ist.

4. System nach Anspruch 1, bei dem das Mittel zum Beschleunigen der Zersetzung ein Kationenaustauscherharz ist.

5. System nach Anspruch 1, bei dem das Mittel zum Beschleunigen der Zersetzung wenigstens ein Bestandteil ist, der Gruppe ausgewählt ist, die aus Adipinsäure, Stearinsäure, Sebacinsäure, Oxalsäure, Itaconsäure, Edetinsäure, Ascorbinsäure, Alginsäure, Asparaginsäure, Sorbinsäure, Weinsäure und Citronensäure besteht.

6. System nach Anspruch 1, bei dem das Mittel zum Beschleunigen der Zersetzung wenigstens ein Bestandteil ist, der aus der Gruppe ausgewählt ist, die aus Natriumhydrogentartrat und Natriumdihydrogencitrat besteht.

7. System nach Anspruch 1, bei dem das Mittel zum Beschleunigen der Zersetzung Natriumhydrogensulfit ist.

8. System nach Anspruch 1, bei dem das Mittel zum Beschleunigen der Zersetzung Glucose ist.

9. System nach Anspruch 1, bei dem einer der Bestandteile der festen Komponente sowohl ein Mittel zum Beschleunigen der Zersetzung des Chlorits als auch ein sauerstoffverbrauchendes Mittel ist.

10. System nach einem der Ansprüche 1, 2 oder 5, bei dem die feste Komponente aus:

einer Säure zum Beschleunigen der Zersetzung des Chlorits unter Bildung von freiem Sauerstoff;

einem Zucker zum Verbrauchen von überschüssigem freiem Sauerstoff aus der Zersetzung des Chlorits, nachdem von der Linse Verunreinigungen entfernt worden sind; und

Polyvinylpyrrolidon als Mittel zum Inhibieren der Zersetzung der Sauer besteht.

11. System nach Anspruch 10, bei dem der Zucker wenigstens ein Bestandteil ist, der aus der Gruppe ausgewählt ist, die aus Glucose, Saccharose und Fructose besteht.

12. Verfahren zum Reinigen von Kontaktlinsen, bei dem eine zu reinigende Linse und ein Reinigungssystem nach Anspruch 1 in einen Behälter eingebracht werden.

13. System nach Anspruch 12, bei dem eine feste Komponente, die aus:

einer Säure zum Beschleunigen der Zersetzung des Chlorits unter Bildung von freiem Sauerstoff;

einem Zucker zum Verbrauchen von überschüssigem freiem Sauerstoff aus der Zersetzung des Chlorits, nachdem von der Linse Verunreinigungen entfernt worden sind; und

Polyvinylpyrrolidon als Mittel zum Inhibieren der Zersetzung der Säure besteht, in den Behälter eingebracht wird.

14. Verfahren nach Anspruch 12, bei dem ein Bestandteil der festen Komponente sowohl als Mittel zum Beschleunigen der Zersetzung des Chlorits als auch als sauerstoffverbrauchendes Mittel wirkt.

15. Ausrüstung zum Reinigen von Kontaktlinsen, enthaltend:

(a) einen Behälter mit 0,0001 bis 5 Masse% eines Chloritsalzes in wäßriger Lösung, das unter Bildung von freiem Sauerstoff zur Entfernung von Verunreinigungen von einer Kontaktlinse zersetzbar ist; und
(b) eine Vielzahl von Packungen, die im voraus abgemessene Mengen
eines Mittels zum Beschleunigen der Zersetzung des Chlorits unter Bildung von freiem Sauerstoff, das aus wenigstens einem Bestandteil besteht, der aus der Gruppe ausgewählt ist, die aus Säuren, Salzen organischer Säuren, Ionenaustauscherharzen, Reduktionsmitteln und Zuckern besteht; und
eines sauerstoffverbrauchenden Mittels zum Verbrauchen von überschüssigem freiem Saauerstoff aus der Zersetzung des Chlorits, nachdem von einer Kontaktlinse, die gereinigt wird, Verunreinigungen entfernt worden sind; und
von Polyvinylpyrrolidon als Mittel zum Inhibieren der Zersetzung des Mittels zum Beschleunigen der Zersetzung des Chlorits enthalten; und
(c) eine Anweisung für den Gebrauch der Ausrüstung zum Reinigen einer Kontaktlinse.

16. Ausrüstung nach Anspruch 15, bei der das Mittel zum Beschleunigen der Zersetzung eine Säure ist und das sauerstoffverbrauchende Mittel ein Zucker ist.

17. Ausrüstung nach Anspruch 16, bei der die Säure aus der Gruppe ausgewählt ist, die aus Adipinsäure, Stearinsäure, Sebacinsäure, Oxalsäure, Itaconsäure, Edetinsäure, Ascorbinsäure, Alginsäure, Asparaginsäure, Sorbinsäure, Weinsäure und Citronensäure besteht.

18. Ausrüstung nach Anspruch 16, bei der der Zucker wenigstens ein Bestandteil ist, der aus der Gruppe ausgewählt ist, die aus Glucose, Saccharose und Fructose besteht.

19. Ausrüstung nach Anspruch 15, bei der eine der Packungen einen Bestandteil enthält, der sowohl ein Mittel zum Beschleunigen der Zersetzung als auch ein sauerstoffverbrauchendes Mittel ist.

20. Ausrüstung nach Anspruch 15, die ferner eine Reinigungsbehälter zum Aufnehmen einer zu reinigenden Kontaktlinse enthält.

**Revendications**

1. Système de nettoyage pour verres de contact, comprenant:
(a) 0,0001 à 5% en poids d'un chlorite en solution aqueuse, capable de se décomposer pour former de l'oxygène libre pour éliminer les impuretés d'un verre de contact;
(b) un constituant solide comprenant:
un agent pour accélérer la décomposition du chlorite pour former de l'oxygène libre, comprenant au moins un élément choisi dans le groupe constitué des acides, des sels d'acides organiques, des résines échangeuses d'ions, des agents réducteurs et des sucres, et un agent consommant de l'oxygène pour consommer l'oxygène libre en excès provenant de la décomposition du chlorite après que les impuretés ont été éliminées du verre de contact nettoyé; et de la polyvinylpyrrolidone comme inhibiteur de décomposition pour cet agent pour accélérer la décomposition du chlorite.

2. Système selon la revendication 1, dans lequel le constituant solide est sous la forme d'un granulé ou d'une poudre.

3. Système selon la revendication 1, dans lequel l'agent pour accélérer la décomposition est au moins un acide organique.

4. Système selon la revendication 1, dans lequel l'agent pour accélérer la décomposition est une résine échangeuse de cations.

5. Système selon la revendication 1, dans lequel l'agent pour accélérer la décomposition est au moins un élément choisi dans le groupe constitué des acides adipique, stéarique, sébacique, oxalique, itaconique, édétique, ascorbique, alginique, aspartique, sorbique, tartrique, et citrique.

6. Système selon la revendication 1, dans lequel l'agent pour accélérer la décomposition est au moins un élèment choisi dans le groupe constitue de l'hydrogénotartrate de sodium et du dihydrogènocitrate de sodium.

7. Système selon la revendication 1, dans lequel l'agent pour accélérer la décomposition est la bisulfite de sodium.

8. Système selon la revendication 1, dans lequel l'agent pour accélérer la décomposition est le glucose.

9. Système selon la revendication 1, dans lequel un des éléments du constituant solide est à la fois un agent pour accélérer la décomposition du chlorite et un agent consommant de l'oxygène.

10. Système selon l'une des revendications 1, 2 ou 5, dans lequel le constituant solide comprend:
un acide pour accélérer la décomposition du chlorite pour former de l'oxygène libre;
un sucre pour consommer l'oxygène libre en excès provenant de la décomposition du chlorite après que les impuretés sont éliminées du verre de contact et:
de la polyvinylpyrrolidone comme inhibiteur de décomposition pour l'acide.

11. Système selon la revendication 10, dans lequel le sucre est au moins un élément choisi parmi le glucose, le saccharose et le fructose.

12. Procédé pour nettoyer des verres de contact, comprenant le fait de placer le verre de contact à nettoyer, et un système de nettoyage selon la revendication 1, dans un récipient.

13. Procédé selon la revendication 12, dans lequel un constituant solide comprenant:
un acide pour accélérer la décomposition du chlorite pour former de l'oxygène;
un sucre pour consommer l'oxygène libre en excès provenant de la décomposition du chlorite après que les impuretés sont éliminées du verre de contact; et

de la polyvinylpyrrolidone comme inhibiteur de décomposition pour l'acide est placée dans le récipient.

14. Procédé selon la revendication 12, dans lequel un élément du constituant solide agit à la fois comme agent d'accélération de la décomposition du chlorite et comme agent consommant l'oxygène.

15. Nécessaire de nettoyage pour verres de contact comprenant:

(a) un récipient avec 0,0001 à 5% en poids d'un chlorite en solution aqueuse capable de se décomposer pour former de l'oxygène libre pour éliminer les impuretés d'un verre de contact; et

(b) plusieurs emballages contenant des quantités mesurées à l'avance de:

un agent pour accélérer la décomposition du chlorite pour former de l'oxygène libre comprenant au moins un élément choisi dans le groupe constitué des acides, des sels d'acides organiques, des résines échangeuses d'ions, des agents réducteurs et des sucres; et un agent consommant de l'oxygène pour consommer l'oxygène libre en excès provenant de la décomposition du chlorite après que les impuretés ont été éliminées du verre de contact nettoyé; et

de la polyvinylpyrrolidone comme inhibiteur de décomposition pour l'agent d'accélérateion de la décomposition du chlorite; et

(c) des instructions pour utiliser le nécessaire pour nettoyer les verres de contact.

16. Nécessaire selon la revendication 15, dans lequel l'agent pour accélérer la décomposition est un acide et l'agent consommant de l'oxygène est un sucre.

17. Nécessaire selon la revendication 16, dans lequel l'acide est choisi dans le groupe constitué des acides adipique, stéarique, sébacique, oxalique, itaconique, édétique, ascorbique, alginique, aspartique, sorbique, tartrique et citrique.

18. Nécessaire selon la revendication 16, dans lequel le sucre est au moins un élément choisi dans le groupe constitué du glucose, du saccharose, et du fructose.

19. Nécessaire selon la revendication 15, dans lequel un des emballages comprend un élément qui est à la fois un agent pour accélérer la décomposition et un agent consommant de l'oxygène.

20. Nécessaire selon la revendication 15, comprenant en outre un récipient de nettoyage pour recevoir le verre de contact à nettoyer.

Fig. 1